# EUROPEAN PATENT APPLICATION

(11) **EP 1 462 143 A1**
(43) Date of publication of application: **29.09.2004**
(21) Application number: 02786008.9
(22) Date of filing: 03.12.2002
(51) Int. Cl.: A61M 25/10

(54) **BALLOON CATHETER AND METHOD OF MANUFACTURING BALLOON CATHETER**

(30) Priority: 07.12.2001 JP 2001375088
(71) Applicant: Kaneka Corporation, Osaka-shi, Osaka 530-0005 (JP)
(72) Inventor: YAMAGUCHI, Youichi, c/o KANEKA CORPORATION, Settsu-shi, Osaka 566 0072 (JP); ISHIBASHI, Takuya, c/o KANEKA CORPORATION, Settsu-shi, Osaka 566 0072 (JP); NISHIDE, Takuji, c/o KANEKA CORPORATION, Settsu-shi, Osaka 566 0072 (JP); TAKATERA, Masayuki, c/o KANEKA CORPORATION, Settsu-shi, Osaka 566 0072 (JP)
(74) Representative: Gille Hrabal Struck Neidlein Prop Roos
(86) International application number: PCT/JP2002/012621
(87) International publication number: WO 2003/047678

(57) **Abstract**

The present invention provides a catheter which makes it possible for a biaxial type balloon catheter having a flexible structure in the joint part between a sleeve on the proximal side of the balloon and a shaft on the distal side thereof, showing no abrupt change in rigidity, reducing the step, and capable of being smoothly advanced through highly constricted and curved blood vessel portions, to be manufactured easily with a high production yield using a small number of manufacturing steps.

The balloon catheter is a biaxial type for medical use, wherein only the inflation lumen of the shaft is removed from the area extending from the joint part between the shaft and the balloon sleeve on the proximal side to the distal end of the shaft, and main walls of the inflation lumen on the distal side from the joint part are formed by the proximal side balloon sleeve.

## Description

### TECHNICAL FIELD

The present invention relates to a balloon catheter used in percutaneous angioplasty (PTA: percutaneous transluminal angioplasty, or PTCA: percutaneous transluminal coronary angioplasty) that is used to dilate and treat constricted portions or blocked portions of coronary arteries, arteries of the extremities, renal arteries, peripheral blood vessels or the like, and a method of manufacturing such a balloon catheter.

### BACKGROUND ART

Balloon catheters used in PTA or PTCA treatments have a balloon on the distal end of a shaft, and most of these catheters are formed from a soft resin.

In the case of PTCA, when the treatment is performed, a guiding catheter is first inserted from a femoral artery, and the tip end of this catheter is positioned at the entrance into the coronary artery via the aorta; then, a guide wire is passed through the site of the pathological change in the constricted portion or blocked portion of the coronary artery or the like. A balloon catheter is inserted along this guide wire, and the balloon is aligned with the site of the pathological change; then, a contrast medium or the like is supplied to the balloon, and the balloon is caused to inflated. Following this dilation therapy of the site of the pathological change, the balloon is deflated and caused to contract, and the dilation catheter is removed from the body.

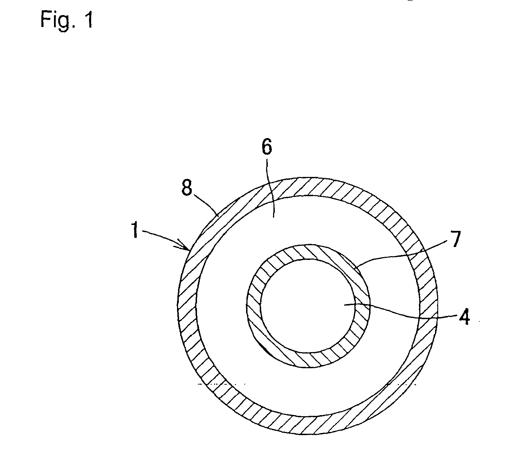

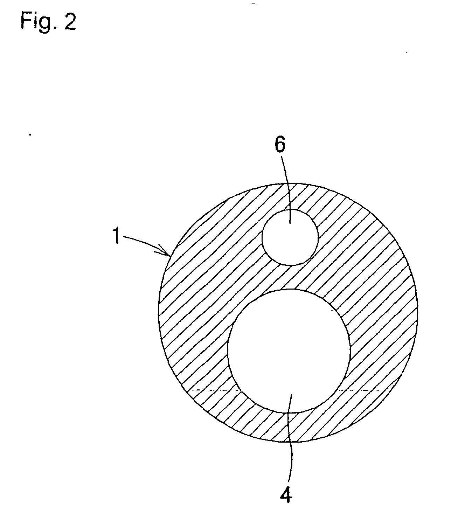

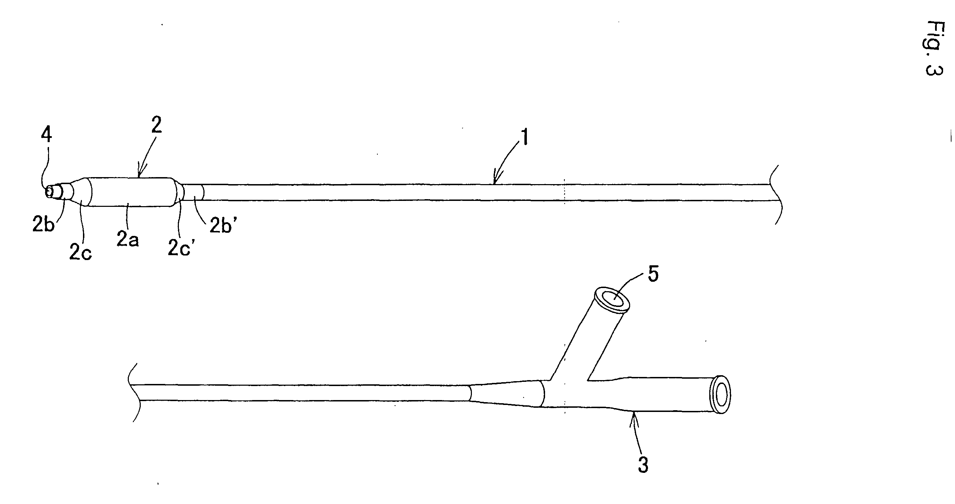

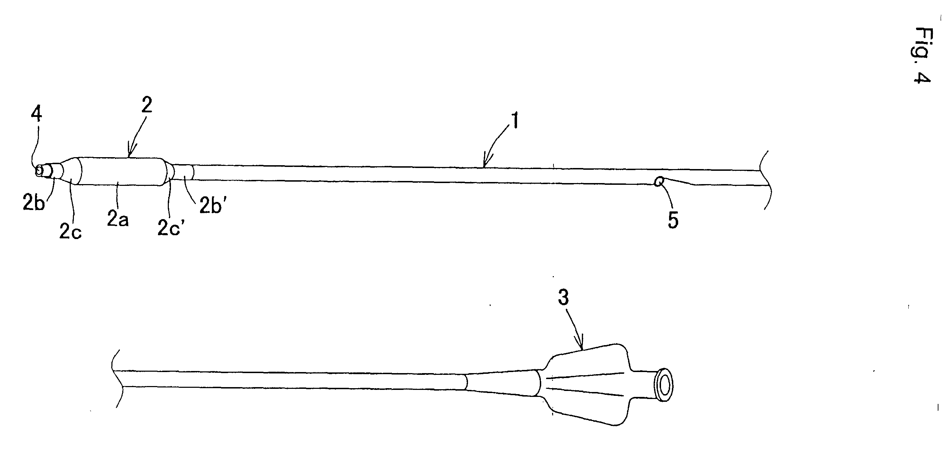

The shaft 1 of such a balloon catheter generally has two lumens. One of these lumens is a lumen 6 (hereafter referred to as the "inflation lumen") which communicates with the balloon, and which allows the passage of a pressurized fluid for the purpose of causing the inflation or contraction of the balloon, and the other lumen is a lumen 4 (hereafter referred to as the "guide wire lumen") which is used to connect the guide wire. Furthermore, a manifold 3 comprising a pressurized fluid supply port that communicates with the inflation lumen 6 is disposed on the proximal portion of the shaft 1. Ordinarily, the shaft 1 is most commonly a shaft with a coaxially disposed double-tube structure in which a guide wire tube 7 that has the guide wire lumen 4 is passed through the interior of an inflation tube 8 that constitutes the inflation lumen 6 (coaxial type, see Fig. 1); however, there are also constructions in which the shaft 1 has an inflation tube and a guide wire tube that are not disposed coaxially, i. e., shafts with a structure in which the inflation lumen 6 and guide wire lumen 4 are formed as an integral unit (biaxial type, see Fig. 2), as in the balloon catheter described in Japanese Patent Application Laid-Open No. 7-178175. Furthermore, balloon catheters can generally be divided into two main categories, i. e., over-the-wire type catheters (Fig. 3) in which the guide wire tube extends for the entire length of the shaft 1 in the axial direction, and monorail type catheters (Fig. 4) in which the guide wire tube is present only in the area extending for a distance of 20 cm to 35 cm from the tip end of the balloon catheter, and a guide wire port 5 is disposed at an intermediate point of the shaft 1.

In balloon catheters used in recent years, there has been a tendency to require catheters that can be used even in highly constricted and curved blood vessel areas. Especially in the case of coronary arteries, which have numerous curved portions, PTCA and PTA balloon catheters that allow the smooth advance of the balloon to sites of pathological changes have been required. In recent years, therefore, the balloon catheters used for PTCA have commonly been constructed using a proximal end tube comprising a relatively hard material in the portion that extends for a distance of approximately 100 cm to 135 cm from the proximal end, and using a distal end tube comprising a relatively soft resin material in the portion that extends for a distance of approximately 20 cm to 35 cm from the distal end. The reason for this is as follows: namely, since the degree of curvature of the aorta through which the proximal end tube passes is small, it is also desirable to use a hard material [for this tube] in order to enhance the pushing force transmission characteristics (pushability); on the other hand, since the degree of curvature of the coronary arteries through which the distal end tube passes is large, it is desirable to use a soft resin material [for this tube] so that the tube can be deformed in conformity to the guide wire.

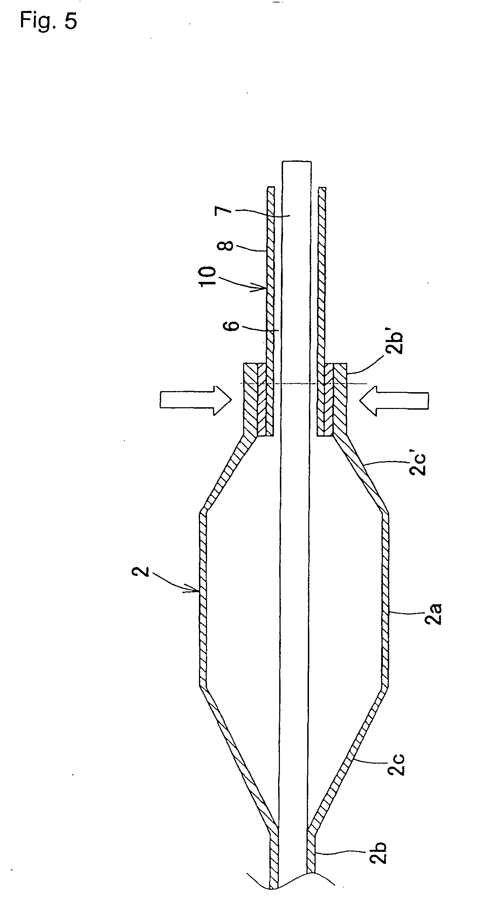

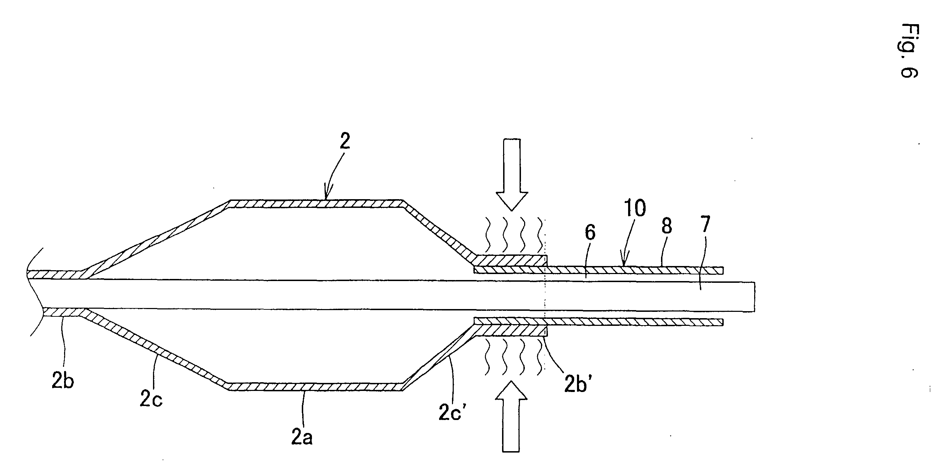

Meanwhile, in the joining of the proximal end balloon sleeve 2b' and the distal side shaft 10, one of these parts is usually fitted and joined with the other part using an adhesive (see Fig. 5), or by means of thermal fusion (see Fig. 6). Such joining techniques are disclosed in Japanese Patent Publication No. 4-670, and a technique using an adhesive is disclosed in Japanese Patent Application Laid-Open No. 6-296693. Furthermore, there are also techniques in which one end of a joint part in which the respective parts are joined by fitting one part over the other is subjected to cutting in order to obtain flexibility of the joint part between the proximal side sleeve of the balloon and the distal side shaft, as disclosed in Japanese Patent Application Laid-Open No. 2000-126299.

However, the following four problems have been encountered in the case of such conventional joining methods and secondary working methods:

First of all, the rigidity varies abruptly in the joint part between the proximal side balloon sleeve and distal side shaft disclosed in Japanese Patent Publication No. 4-670 or Japanese Patent Application Laid-Open No. 6-296693, so that the-pressing force from the manifold to which the proximal end of the shaft is connected is not transmitted beyond the joint part. In other words, since the proximal side sleeve of the balloon and the distal side shaft are joined by one part being fit over the other, the joint part has a two-layer structure, and is therefore extremely hard; furthermore, since a three-layer structure that includes the adhesive layer is formed, this joint part becomes even harder. Consequently, since the distal end tube is flexible and the joint part is harder than the distal end tube, a place is created where there is an abrupt change in rigidity. Accordingly, when the balloon catheter is passed through highly constricted and curved blood vessel portions, the pressing force that is applied from the proximal side of the shaft is not transmitted to the distal end of the shaft beyond the point where the rigidity changes abruptly, and in the worst case, the shaft is bent at the point where the rigidity changes abruptly. In such cases, the pushability of the dilation catheter shows an extreme drop.

Secondly, the area of the two-layer or three-layer structure in the joint part becomes extremely hard, and in cases where this portion is long, this portion has a rigidity that tends to maintain a rectilinear shape, so that the property whereby the balloon catheter smoothly bends along the blood vessel (trackability) shows an extreme drop. As a result, for example, when the joint part attempts to pass through the end portion of the guiding catheter, the operator experiences a large resistance, so that a major inconvenience is created.

Third, since a portion with a two-layer or three-layer structure is created in the joint part, the external diameter of this portion is increased, and a large step is generated. When the external diameter is increased and a large step is generated in the joint part, there is a danger that the balloon catheter will become caught on the blood vessel walls or the interior of the guiding catheter when the balloon catheter is advanced or retracted, so that it becomes difficult to pull the balloon catheter out. Furthermore, since the balloon joint part passes through highly constricted and curved blood vessel portions even among blood vessels, these problems have a great effect on the ability of the balloon catheter to pass through.

Fourth, if the joint part is subjected to cutting working by the working method disclosed in Japanese Patent Application Laid-Open No. 2000-126299, the following problem arises: namely, working defects such as surface roughness, nap and the like are continuously generated on the surfaces of the worked portions, so that the production yield in the manufacturing process is extremely poor. Moreover, the working process must be performed following the joining, so that the manufacture of the catheter is costly and time-consuming.

### DISCLOSURE OF THE INVENTION

In light of the problems, it is an object of the present invention to provide a balloon catheter which makes it possible for a biaxial type balloon catheter that has a flexible structure in the joint part between the balloon on the proximal side and the shaft on the distal side, that shows no abrupt change in rigidity, that reduces the step, and that can be smoothly advanced through highly constricted and curved blood vessel portions, to be manufactured easily with a high production yield using a small number of manufacturing steps.

The balloon catheter of the present invention is a balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and the shaft, and only the guide wire lumen is passed through the balloon and extended toward the distal end, characterized in that the end surface of the sleeve and the sectional surface that is newly created by the removal of the inflation lumen are caused to abut each other and are joined. As a result of the adoption of such a structure, the joint part becomes flexible and the step is reduced, so that the problems are solved.

Furthermore, by using a joint part constructed from materials that can be joined by fusion as the joint part, and joining these materials by fusion, it is possible to obtain strength during pressing under the high pressure of the balloon; accordingly, the use of such materials is desirable. Furthermore, in order to increase the cross-sectional area of the inflation lumen in the direction of diameter, it is desirable to use a single inflation lumen.

Moreover, the present invention provides a balloon catheter manufacturing method in which the end surface of the sleeve and the sectional surface that is newly created by the removal of the inflation lumen are caused to abut each other and are joined in a balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and the shaft, and only the guide wire lumen is passed through the balloon and extended toward the distal end. Furthermore, in the balloon catheter manufacturing method of the present invention, the external diameter and shape of the balloon catheter can be formed by using a core material when the joint part and the inflation lumen on the distal side from the joint part are formed. This balloon catheter manufacturing method of the present invention allows a balloon catheter that is flexible and that has no step difference to be manufactured easily with a high production yield using a small number of manufacturing method steps.

### BRIEF DESCRIPTION OF THE DRAWINGS

Fig. 1 is a schematic sectional view which shows a coaxial type shaft in a common balloon catheter;
Fig. 2 is a schematic sectional view which shows a biaxial type shaft in a common balloon catheter;
Fig. 3 is a schematic side view which shows an over-the-wire type system in a common balloon catheter;
Fig. 4 is a schematic side view which shows a monorail type system in a common balloon catheter;
Fig. 5 is a schematic sectional view which shows a joint part using an adhesive in the joining of the balloon sleeve on the proximal side and the shaft on the distal side;
Fig. 6 is a schematic sectional view which shows a joint part using fusion in the joining of the balloon sleeve on the proximal side and the shaft on the distal side;
Fig. 7 is a schematic side view which shows one embodiment of the biaxial type balloon catheter of the present invention;
Fig. 8 is a schematic sectional view of one embodiment of the biaxial type balloon catheter of the present invention, showing the abutment and joining of the end portion of a proximal side balloon sleeve with a perpendicular shape to the end portion of a distal side inflation lumen which is perpendicular to the axial direction of the balloon catheter;
Fig. 9 is a schematic sectional view of one embodiment of the biaxial type balloon catheter of the present invention, showing the abutment and joining of the end portion of a proximal side balloon sleeve with a tapered shape to the end portion of a distal side inflation lumen which has a tapered shape with respect to the axial direction of the balloon catheter;
Fig. 10 shows one example of the shaft of the biaxial type balloon catheter of the present invention;
Fig. 11 is a schematic sectional view of the biaxial type catheter of the present invention, showing the end portion of a distal side shaft part removed in a tapered shape with respect to the axial direction of the balloon catheter;
Fig. 12 is a schematic sectional view of the biaxial type balloon catheter of the present invention, showing a joining method using a core material in which the end portion of a proximal side balloon sleeve that has a perpendicular shape is placed against the end portion of a distal side inflation lumen that has a tapered shape with respect to the axial direction of the balloon catheter; and
Fig. 13 is a schematic sectional view of a conventional biaxial type balloon catheter.

### BEST MODE FOR CARRYING OUT THE INVENTION

Various embodiments of the balloon catheter of the present invention will be described below with reference to the attached figures.

The balloon catheter of the present invention relates to a balloon catheter which is constructed from a balloon 2 that has a proximal side sleeve 2b', generally a balloon 2 which has a straight tubular part 2a, a proximal side tapered part 2c' and distal side tapered part 2c which are adjacent to both ends of the strait tubular part, and which gradually decrease in diameter, and a proximal side sleeve 2b' and distal side sleeve 2b which are adjacent to these tapered parts, and a shaft 1 which has an inflation lumen 6 and a guide wire lumen 4 as integral units, generally a biaxial type distal side shaft 10. This balloon catheter may be either an over-the-wire type catheter, or a monorail type catheter. Furthermore, other constructions likewise do not restrict the effect of the invention.

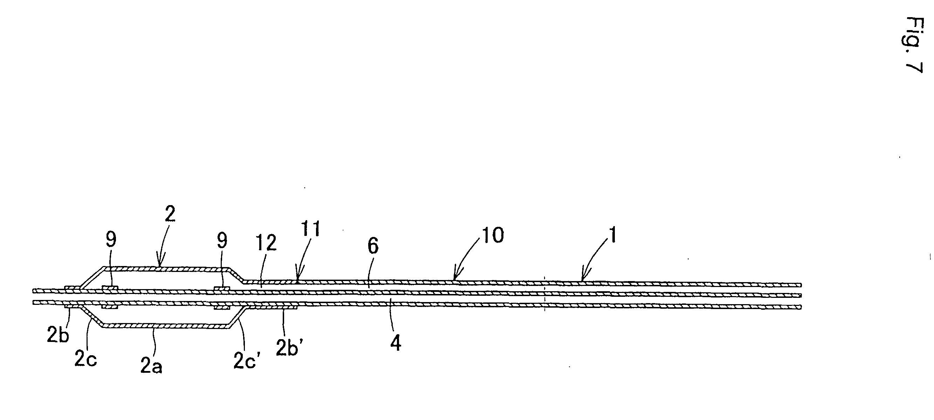

Fig. 7 shows on embodiment of the present invention. The joining of the distal side shaft 10 and the sleeve 2b' on the proximal side of the balloon in the present invention is characterized by the following: namely, after the inflation lumen is removed by removing the portion of the wall thickness of the inflation lumen 6 in a specified section so that the portion of the wall thickness that forms the guide wire lumen 4 of the distal side shaft 10 is left, the proximal side balloon sleeve 2b' is caused to cover the remaining guide wire lumen 4, and is joined to this guide wire lumen 4; furthermore, the end surface of the sleeve 2b' and the sectional surface newly generated by the removal of the inflation lumen 6 are caused to abut [each other] and are joined. As a result, a balloon catheter can be provided which has a flexible structure in the joint part 11 between the proximal side balloon sleeve 2b' and the distal side shaft 10, which shows no abrupt changes in rigidity, which has the reduced step, and which can [therefore] be passed through highly constricted and curved blood vessel portions. In order to obtain a flexible structure, it is desirable that the wall thickness portion of the inflation lumen be completely removed; however, a structure in which a portion of this wall thickness portion is allowed to remain may also be used in order to obtain the required rigidity or for other purposes.

The term "joint part 11" used here refers to the portion extending around the circumference where the distal side shaft 10 and balloon proximal side sleeve 2b' contact each other and are fastened to each other. Furthermore, the joint part 11 is divided into two main parts on the basis of the joint configuration. One of these parts is positioned on the side of the inflation lumen 6 on the circumference, and is joined mainly by the abutment of the end surface of the sleeve 2b' and the sectional surface (which is perpendicular or tapered with respect to the axis of the balloon catheter) that is newly generated by the removal of the inflation lumen 6; in the present application, this joint part is referred to as the "inflation lumen side joint part". The other joint part is positioned on the side of the guide wire lumen 4 on the circumference, and is joined with the surface of the distal side shaft 10 being covered by the balloon proximal side sleeve 2b'; in the present application, this joint part is referred to as the "guide wire lumen side joint part".

In order to obtain a balloon catheter that can advance smoothly through highly constricted and curved blood vessel portions, it is desirable that the maximum external diameter of the joint part 11 be set at a diameter that is the same or smaller than the maximum diameter of the balloon sleeve 2b' in the range adjacent to the joint part 11, or the maximum diameter of the shaft 10 in the range adjacent to the joint part 11. Here, the term "range adjacent to" refers to a distance of approximately 10 cm from the joint part 11. However, even if the maximum external diameter of the joint part 11 cannot be made smaller than the maximum diameter of the balloon sleeve 2b' or the maximum diameter of the shaft 10, this can be used for the purpose of merely reducing the step difference.

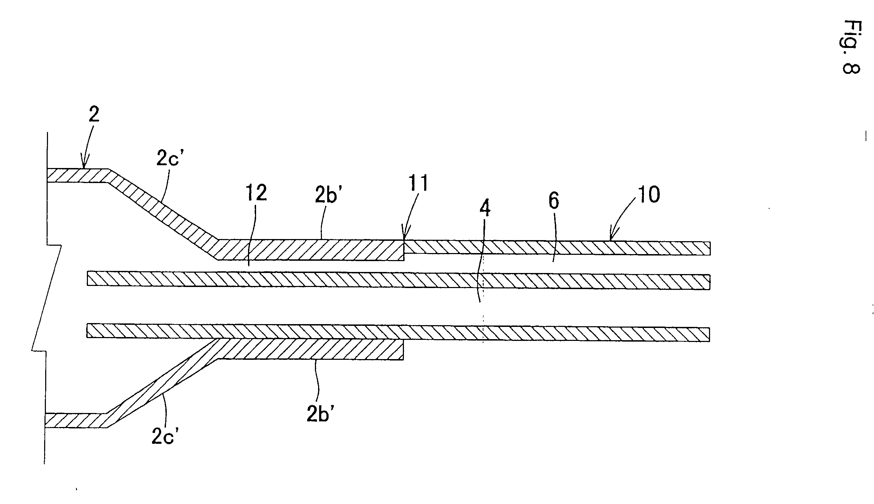

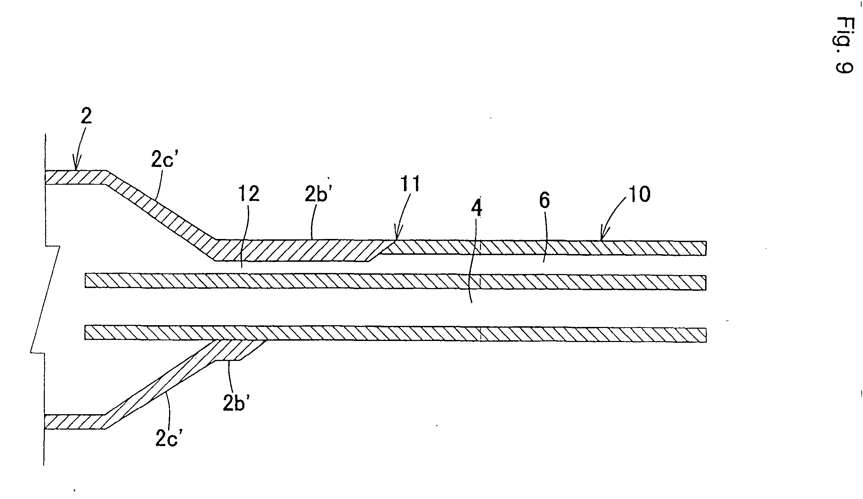

The method used to join the proximal side balloon sleeve 2b' to the shaft 10 may be either of the joining methods shown in Figs. 8 and 9. For example, a method in which joining is performed in a state in which the proximal side balloon sleeve 2b' with an end surface perpendicular to the axial direction of the balloon catheter is caused to abut against a sectional surface formed by removing the inflation lumen 6 so that this sectional surface is perpendicular to the axial direction of the balloon catheter as shown in Fig. 8 may be used, or a method in which joining is performed in a state in which the proximal side balloon sleeve cut in a tapered shape is caused to abut against the end portion of the distal side inflation lumen removed in a tapered shape as shown in Fig. 9 may be used. In cases where the joining method shown in Fig. 8 is used, the external diameter of the end portion of the joint part 11 can be reduced, while in cases where the joining method shown in Fig. 9 is used, the area of bonding or fusion of the end portion of the joint part 11 can be increased. These joining methods can be selected and used in accordance with the application involved. Furthermore, by cutting the end portion of the proximal side balloon sleeve 2b' in a curved shape or step-form shape instead of the rectilinear shape shown in Figs. 8 and 9, it would also be possible to join the parts in the inflation lumen side joint part with a sectional surface that is perpendicular to the axial direction of the balloon 2 as shown in Fig. 8, and to join the parts in the wire guide lumen side joint part so that the overlapping width of the shaft 10 and balloon sleeve 2b' is reduced as shown in Fig. 9. In this case, the joint part 11 can be formed with greater flexibility. Furthermore, various cut shapes may be used as necessary. Moreover, a relatively flexible joint part 11 is obtained even if the inflation lumen side joint part is formed with overlapping, [as long as] the joining margin of the proximal side balloon sleeve 2b' and the shaft 10 is 4 mm or less, preferably 2 mm or less. The joining method shown in Fig. 9 is especially desirable for forming the joint part 11 with flexibility, and for obtaining an even high production efficiency.

As long as the balloon catheter is a balloon catheter in which the maximum length for which the proximal side balloon sleeve 2b' forms the inflation lumen 6 from the inflation lumen side joint part toward the distal end, i. e., the length from the inflation lumen side joint part to the proximal end of the proximal side tapered part 2c', is a length of 1 mm to 200 mm, any lumen length may be used. However, from the standpoint of simplicity and ease of the bonding method during production, a balloon catheter which has an inflation lumen length of 2 mm to 10 mm is desirable. If the maximum length for which the proximal side balloon sleeve 2b' forms the inflation lumen from the inflation lumen side joint part toward the distal end is less than 2 mm, there is a very real possibility of damage to the balloon during the joining of the balloon 2 and the shaft 10, and if this length is less than 1 mm, it becomes difficult to achieve this joining without damaging the balloon. On the other hand, if this length is greater than 10 mm, since the proximal side balloon sleeve 2b' is thin in most cases, it becomes difficult to obtain good pushability, and if this length exceeds 200 mm, there is an occurrence of kinking and the like.

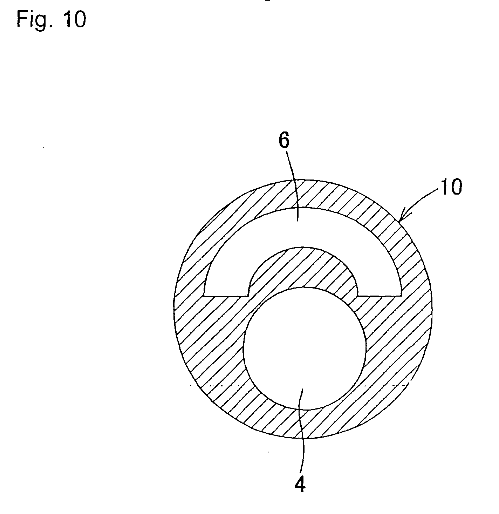

Fig. 10 shows an embodiment of the present invention; this figure is a sectional view of the shaft 10. The cross-sectional shape of the shaft 10 may be any shape such as round, oval, square or the like; however, a round shape is desirable from the standpoint of preventing damage to blood vessels.

The cross-sectional shape and number of inflation lumens 6 formed in the shaft may be any shape and number; however, from the standpoint of reducing pressure loss, it is desirable to form a single lumen with a round shape.

The cross-sectional shape of the guide wire lumen 4 formed in the shaft 10 may be any shape; however, a round shape is desirable from the standpoint of guide wire sliding characteristics. The joining method used to join the shaft 10 and the proximal side balloon sleeve 2b' may be any joining method such as a joining method using an adhesive, a joining method using fusion or the like; however, a joining method using fusion is desirable from the standpoints of the processing accuracy and processing yield of the joint part 11.

The fusion connection method used for the joint part 11 may be any type of fusion method such as hot air welding, ultrasonic fusion, laser fusion, fusion by means of chemicals or the like; however, a joining method using thermal fusion is desirable from the standpoint of the ease of operation and safety of the worker and the like.

Any method may be used as the method of forming the shape and internal diameter of the joint part 11 and the inflation lumen 12 on the distal side from this joint part; however, a method using a core material 13, 14 is desirable from the standpoints of ease of working and processing accuracy, and it is desirable that the outside part of the core material 13, 14 be coated in order to facilitate removal following working by means of an adhesive or fusion.

Furthermore, there are no restrictions on the material of the joint part 11, as long as joining by means of fusion is possible. Specifically, the materials of the proximal side balloon sleeve 2b' and shaft 10 that are used may be the same or different materials, or may be multilayered materials in which different materials are laminated, as long as these materials can be fused. For example, in the case of a PTCA balloon catheter, the balloon 2 is flexible and thin, and a high strength performance is required. Accordingly, a polyolefin, polyolefin elastomer, polyester, polyester elastomer, polyamide, polyamide elastomer, polyurethane or polyurethane elastomer is desirable. The material of the shaft 10 may be the same material or a different material, as long as this material can be fused with the materials; however, from the standpoint of the fused strength of the joint part 11 and the like, it is desirable to use the same material or an elastomer of the same material for the shaft 10. Accordingly, in cases where the balloon 2 and proximal side balloon shaft 2b' are formed from a polyamide material, it is desirable to use a shaft 10 in which a single layer or at least one layer of a multilayered material is formed from a polyamide or polyamide elastomer.

### Examples

More concrete examples and comparative examples of the present invention will be described in detail below; however, the present invention is not limited by these examples.

### (Example 1)

A tubular parison (internal diameter 1.10 mm, external diameter 2.30 mm) was manufactured by an extrusion molding method using a polyamide elastomer (commercial name: PEBAX7233SA01, manufactured by Elf Atochem Co.). Next, a balloon 2 in which the external diameter of the straight tubular part was 7.0 mm was manufactured by a biaxially stretching blow-molding method using this parison. A shaft 10 (external diameter 1.65 mm, internal diameter of guide wire lumen 0.95 mm, internal diameter of inflation lumen 0.40 mm) with the cross-sectional shape shown in Fig. 2 was manufactured by extrusion molding using a polyamide elastomer (commercial name: PEBAX7233SA01, manufactured by Elf Atochem Co.).

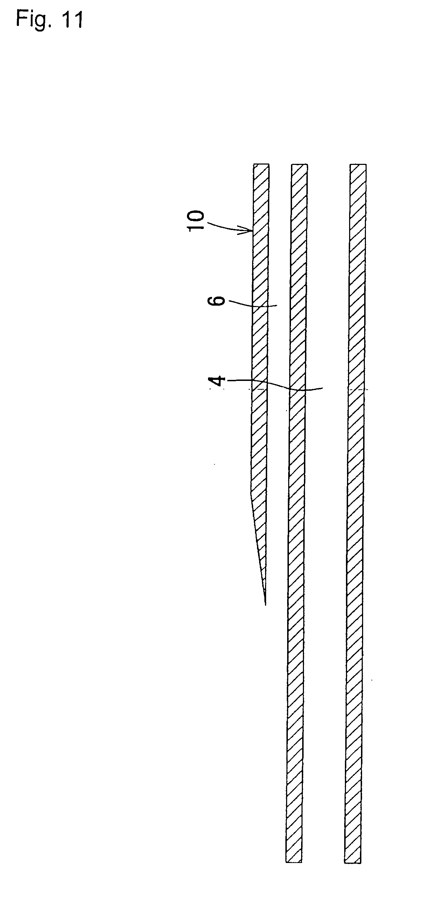

Next, the wall thickness portion of the inflation lumen 6 of the shaft 10 was cut in a tapered shape and removed as shown in Fig. 11 from a position located 8 mm from the distal end, with the wall thickness portion of the guide wire lumen 4 left in place.

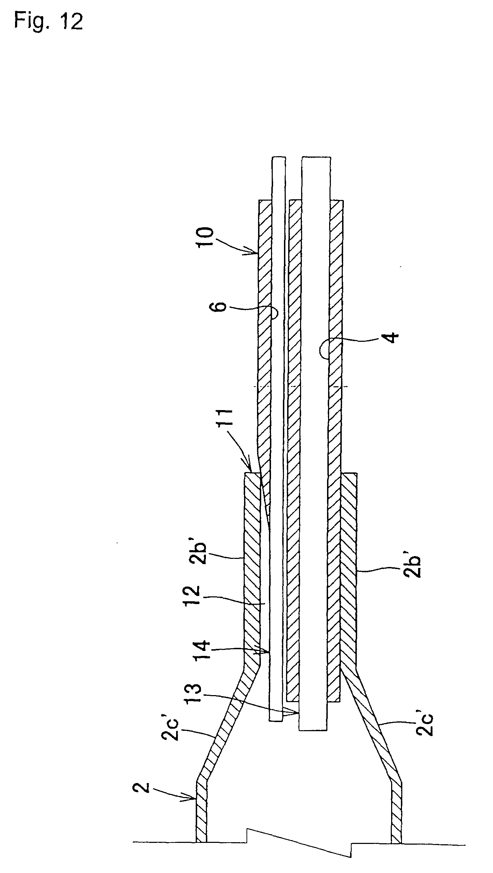

Then, as is shown in Fig. 12, a core material 13 (external diameter 0.94 mm) was disposed in the guide wire lumen 4 of the shaft 10, and a core material 14 (external diameter 0.40 mm) as disposed in the inflation lumen 6, so that a length allowing sufficient passage was maintained.

Next, after the proximal side balloon sleeve 2b' was perpendicularly cut (with respect to the axial direction of the balloon catheter) at a position separated from the beginning portion of the proximal side tapered part 2c' by a distance of 8 mm in the proximal direction, the end portion of the proximal side balloon sleeve 2b' and the distal end portion of the shaft 10 were disposed as shown in Fig. 12 and covered with a heat-shrink tube (internal diameter 1.70 mm); then, the sample of this example was manufactured by hot air welding with the values of the hot air welding machine set at 260°C, 5 L/min.

### (Example 2)

The sample of this example was manufactured by the same method as that described in Example 1, except for the fact that the proximal side balloon sleeve 2b' was cut in a tapered shape (with respect to the axial direction of the balloon catheter) at a position separated from the beginning portion of the proximal side tapered part 2c' by a distance of 8 mm in the proximal direction.

### (Example 3)

The sample of this example was manufactured by the same method as that described in Example 1, except for the fact that the wall thickness portion of the inflation lumen was cut perpendicularly with respect to the axial direction of the balloon catheter and removed (as shown in Fig. 8) for a distance of 8 mm from the distal end, with the wall thickness portion of the wire guide catheter 4 left in place.

### (Comparative Example 1)

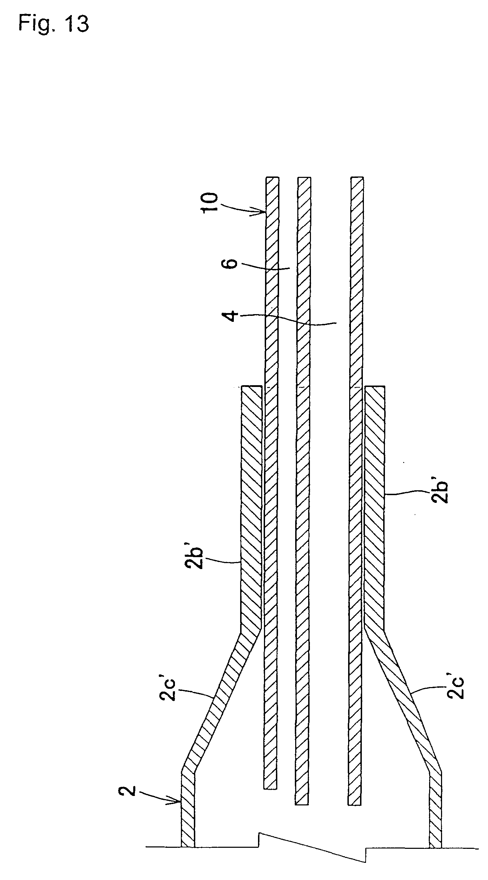

The sample of this [comparative] example was manufactured by the same method as that described in Example 1, except for the fact that the wall thickness portion of the inflation lumen 6 of the shaft was left without being cut, and the proximal side balloon sleeve 2b' was covered with a heat-shrink tube (internal diameter 2.38 mm) after being disposed on the shaft 10 so as to fit over the shaft 10 as shown in Fig. 13.

Three samples each of Examples 1 through 3 and Comparative Example 1 were evaluated by the following method.

### (Evaluation)

The maximum external diameter of the joint part 11 was measured using a laser external diameter measuring device (commercial name: LS-3100, manufactured by KEYENCE). As is shown in Table 1, the evaluation results were as follows: namely, a smaller diameter was obtained in the case of the external diameters of the joint parts of the examples than in the case of the external diameter of the joint part of the comparative example. Furthermore, when a check was made by tactile sensory perception, the examples were all superior to the comparative examples in terms of flexibility and continuity of rigidity. Moreover, [in the examples,] the working of the joint part was relatively easy and simple, and the processing yield was also extremely good.

**Table 1**

| Measurement results for maximum external diameter of joint part. | | | | |
|---|---|---|---|---|
| | Comparative Example 1 | Embodiment 1 | Embodiment 2 | Embodiment 3 |
| 1 | 1.97 | 1.69 | 1.63 | 1.62 |
| 2 | 1.99 | 1.67 | 1.65 | 1.63 |
| 3 | 1.98 | 1.66 | 1.64 | 1.60 |
| *Units are all mm (millimeters). | | | | |

### INDUSTRIAL APPLICABILITY

As was described above, [the present invention] is a balloon catheter in which at least the distal portion of the balloon catheter is formed using a shaft that has an inflation lumen and a guide wire lumen as integral parts, and a balloon which has a sleeve on at least the distal end, and in which only the inflation lumen on the distal end is removed, and only the guide wire lumen is passed through the balloon and extended toward the distal end, from the joint part between the sleeve on the proximal side of the balloon and the shaft, wherein the end surface of the sleeve and the sectional surface that is newly created by the removal of the inflation lumen are caused to abut each other and are joined.
Accordingly, [this catheter] has a flexible structure in the joint part between the balloon on the proximal side and the shaft on the distal side. Furthermore, the catheter shows no abrupt change in rigidity, has the reduced step, and can be smoothly advanced through highly constricted and curved blood vessel portions. Moreover, since a core material is used in the manufacturing method, the catheter can easily be manufactured with a high production yield using a small number of manufacturing steps.

## Claims

1. A balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and said shaft, and only the guide wire lumen is passed through the balloon and extended toward the distal end, wherein the end surface of the sleeve and the sectional surface that is newly created by the removal of the inflation lumen are caused to abut each other and are joined.

2. The balloon catheter according to claim 1, **characterized in that** the maximum external diameter of the joint part between the sleeve on the proximal side of the balloon and said shaft is the same as or smaller than the maximum diameter of the proximal side balloon sleeve adjacent to said joint part, or the maximum diameter of said shaft adjacent to said joint part.

3. The balloon catheter according to claim 1 or claim 2, **characterized in that** the maximum external diameter of the joint part between the sleeve on the proximal side of the balloon and said shaft is the same as or smaller than the maximum diameter of the proximal side balloon sleeve adjacent to said joint part, and the maximum diameter of said shaft adjacent to said joint part.

4. The balloon catheter according to any one of claims 1 through 3, wherein said inflation lumen is a single lumen.

5. The balloon catheter according to any one of claims 1 through 4, wherein the joint part between the sleeve on the proximal side of the balloon and said shaft is joined by fusion.

6. The balloon catheter according to any one of claims 1 through 5, wherein said sleeve on the proximal side of the balloon and said shaft are constructed from materials that can be joined by fusion.

7. The balloon catheter according to any one of claims 1 through 6, wherein the maximum length of the balloon sleeve forming the inflation lumen toward the distal end from the joint part on the side of the inflation lumen is a length of 1 mm to 200 mm.

8. The balloon catheter according to claim 7, wherein the maximum length of the balloon sleeve forming the inflation lumen toward the distal end from the joint part on the side of the inflation lumen is a length of 2 mm to 10 mm.

9. A balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and said shaft, and only the guide wire lumen is passed through the balloon and extended toward the distal end, wherein the area in the vicinity of the end surface of the sleeve and the area in the vicinity of the sectional surface that is newly created by the removal of the inflation lumen are joined so that these parts overlap with a joining margin of 4 mm or less, and the length for which the balloon sleeve alone forms the inflation lumen toward the distal end from the joint part on the side of the inflation lumen is a length of 1 mm to 200 mm.

10. The balloon catheter according to claim 9, wherein the length for which the balloon sleeve alone forms the inflation lumen toward the distal end from the joint part on the side of the inflation lumen is a length of 2 mm to 10 mm.

11. A balloon catheter manufacturing method in which the end surface of the sleeve and the sectional surface that is newly created by the removal of the inflation lumen are caused to abut each other and are joined in a balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and said shaft, and only the guide wire lumen is passed through the balloon and extended toward the distal end.

12. A balloon catheter manufacturing method in which the area in the vicinity of the end surface of the sleeve and the area in the vicinity of the sectional surface that is newly created by the removal of the inflation lumen are joined so that these parts overlap with a joining margin of 4 mm or less, and the length for which the balloon sleeve alone forms the inflation lumen toward the distal end from the joint part on the side of the inflation lumen is set at 1 mm to 200 mm, in a balloon catheter in which at least the distal portion of the balloon catheter is formed using a shaft that has an inflation lumen and a guide wire lumen as integral parts, and a balloon which has a sleeve on at least the proximal side, and in which only the inflation lumen on the distal end is removed from the joint part between the sleeve on the proximal side of the balloon and said shaft, and only the guide wire lumen is passed through the balloon and extended toward the distal end.

13. The balloon catheter manufacturing method according to claim 12, wherein the length for which the balloon sleeve alone forms the inflation lumen toward the distal end from the joint part on the side of the inflation lumen is set at 2 mm to 10 mm.

14. The balloon catheter manufacturing method according to any one of claims 11 through 13, **characterized in that** an internal diameter and shape of said joint part and the inflation lumen on the distal side from said joint part are secured using a core material.
